# EUROPÄISCHE PATENTSCHRIFT

(11) **EP 0 584 115 B1**
(45) Veröffentlichungstag und Bekanntmachung des Hinweises auf die Patenterteilung: **21.08.1996**
(21) Anmeldenummer: 92909338.3
(22) Anmeldetag: 04.05.1992
(51) Int. Cl.: C07F 9/553, A61K 31/675

(54) **ARZNEIMITTEL ENTHALTEND AZACYCLODIPHOSPHONSÄUREDERIVATE, NEUE AZACYCLODIPHOSPHONSÄUREDERIVATE SOWIE VERFAHREN ZU DEREN HERSTELLUNG**
MEDICAMENTS CONTAINING AZACYCLODIPHOSPHONIC ACID DERIVATIVES, NOVEL AZACYCLODIPHOSPHONIC ACID DERIVATIVES AND PROCESS FOR PRODUCING THEM
MEDICAMENTS CONTENANT DES DERIVES DE L'ACIDE AZACYCLODIPHOSPHONIQUE, NOUVEAUX DERIVES DE L'ACIDE AZACYCLODIPHOSPHONIQUE ET LEUR PROCEDE DE FABRICATION

(30) Priorität: 04.05.1991 DE 4114586
(43) Veröffentlichungstag der Anmeldung: 02.03.1994
(73) Patentinhaber: BOEHRINGER MANNHEIM GMBH, 68298 Mannheim (DE)
(72) Erfinder: ZILCH, Harald, D-6800 Mannheim 31 (DE); ESSWEIN, Angelika, D-7700 Singen (DE); BAUSS, Frieder, D-6715 Lambsheim (DE)
(74) Vertreter: Weber, Manfred, Dr.
(86) Internationale Anmeldenummer: EP9200967
(87) Internationale Veröffentlichungsnummer: WO9219628

(56) Entgegenhaltungen:
- EP-A- 0 332 068
- DE-A- 2 541 981
- FR-A- 2 245 372

## Beschreibung

Die vorliegende Erfindung betrifft Arzneimittel, die Azacyclodiphosphonsäurederivate enthalten, neue Azacyclodiphosphonsäurederivate sowie Verfahren zu deren Herstellung.

In der DE-A-2611768 und in der DE-A-2610678 werden Azacycloalkandiphosphonsäuren als Bestandteile von photographischen Farbentwicklermischungen und Pigmentaufschlämmungen als gute calciumkomplexbildner beschrieben. Namentlich aufgeführt sind die Azacycloheptan(2,2)diphosphonsäure, Azacylopentan(2,2)diphosphonsäure und die Azacyclotridecan(2,2)diphosphonsäure und deren Salze.

In der DE-A-2541981/DE-A-2343196 sind Verfahren zur Herstellung dieser Azacycloalkandiphosphonsäuren genannt.

In der EP-A-0 332 068 ist ein Verfahren der Herstellung von Azacycloalkandiphosphonsaüren beschrieben, wobei namentlich Verbindungen mit n = 3, 4, 5 und 6 aufgeführt werden

Es wurde nun gefunden, daß Azacycloalkandiposphonsäure-Derivate auch eine ausgezeichnete Wirkung auf den Calciumstoffwechsel zeigen und damit zur breiten Behandlung von Calciumstoffwechselstörungen geeignet sind. Sie lassen sich vor allem sehr gut dort einsetzen, wo der Knochenauf- und abbau gestört ist, d.h. sie sind geeignet zur Behandlung von Erkrankungen des Skelettsystems wie z.B. Osteoporose, Morbus Paget, Morbus Bechterew u.a..

Aufgrund dieser Eigenschaften finden sie aber auch Verwendung in der Therapie der Urolithiasies und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose.

Gegenstand der vorliegenden Erfindung sind demnach Arzneimittel, die Diphosphonate der allgemeinen Formel I sowie deren pharmakologisch unbedenkliche Salze 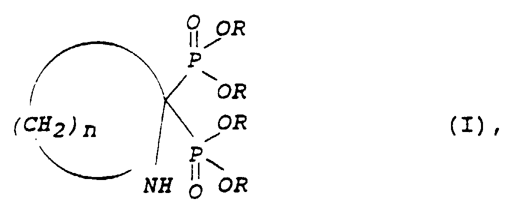 in der
R = Wasserstoff oder C₁-C₄-Alkyl,
und
n = 7-16
bedeuten, enthalten und deren Verwendung zur Behandlung von Knochenstoffwechselstörungen.

Der Rest R bedeutet vorzugsweise Wasserstoff oder den Methyl-, Ethyl- oder Isobutylrest.
n ist vorzugsweise 8, 9, 10, 11, 12.

Gegenstand der Erfindung sind auch neue Verbindungen der allgemeinen Formel I' 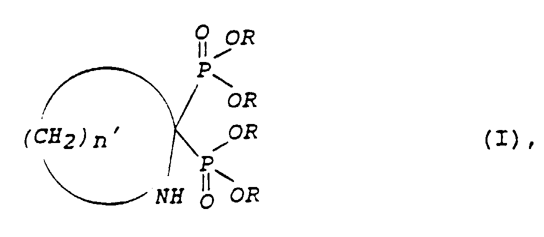 in der
R Wasserstoff oder C₁-C₄-Alkyl und
n' = 7-16
bedeuten, sowie deren pharmakologisch unbedenkliche Salze,
wobei n' nicht gleich 11 sein darf.

Der Rest R bedeutet vorzugsweise Wasserstoff oder der Methyl-, Ethyl- oder Isobutylrest.
n' ist vorzugsweise 8, 9, 10 oder 12.

Verbindungen der allgemeinen Formel I werden nach an sich bekannten Verfahren hergestellt. Dies geschieht vorzugsweise durch Umsetzung eines Lactams der allgemeinen Formel II 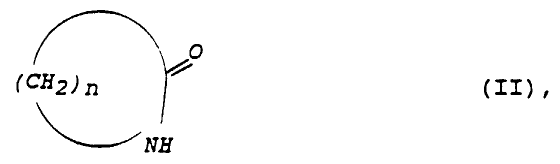 in der n die oben angegebene Bedeutung hat, mit einem Gemisch aus phosphoriger Säure oder Phosphorsäure und einem Phosphorhalogenid oder Phosphorylhalogenid, oder auch indem man das Phosphorhalogenid allein in Gegenwart von Wasser zur Reaktion bringt und anschließend zur freien Diphosphonsäure hydrolisiert, oder gewünschtenfalls die isolierten Verbindungen der allgemeinen Formel I in deren Ester bzw. pharmakologisch unbedenkliche Salze überführt.

Die bei dem Herstellungsverfahren eingesetzten Lactame der allgemeinen Formel II werden mit 1-5, vorzugsweise 2-3 mol phosphoriger Säure oder Phosphorsäure und 1-5, vorzugsweise 2-3 mol Phosphorylhalogenid, Phosphortrihalogenid oder Phosphorpentahalogenid versetzt und bei 80-100°C, vorzugsweise 100°C, unter Inertgasatmosphäre, vorzugsweise Stickstoffatmosphäre, zur Reaktion gebracht. Bei den Phosphor- oder Phosphorylhalogeniden handelt es sich vorzugsweise um die Chloride bzw. Bromide. Man kann die Reaktion auch in Gegenwart von Verdünnungsmitteln, wie Halogenkohlenwasserstoffen, insbesondere Chlorbenzol, Tetrachlorethan oder auch Dioxan gegenbenenfalls unter Zusatz von Wasser, durchführen. Die anschließende Hydrolyse erfolgt durch Erhitzen mit Wasser, zweckmäßiger jedoch mit halbkonzentrierter Chlor- oder Bromwasserstoffsäure.

Die freie Diphosphonsäuren der allgemeinen Formel I können durch Erhitzen mit Orthoameisensäurealkylestern in die entsprechenden Tetraalkylester überführt werden. Die Verseifung zu Diestern geschieht in der Regel dadurch, daß man den Tetraalkylester mit einem Alkalihalogenid, vorzugsweise Natriumjodid in einem geeigneten Lösungsmittel wie z.B. Aceton bei Zimmertemperatur behandelt.

Hierbei entsteht das symmetrische Diester/Dinatriumsalz, das gegebenenfalls durch einen sauren Ionenaustauscher in die Diester/Disäure umgewandelt werden kann. Die Verseifung der Ester zu freien Diphosphonsäuren geschieht in der Regel durch Kochen mit Chlor- oder Bromwasserstoffsäure. Man kann jedoch auch eine Spaltung mit Trimethylsilylhalogenid, vorzugsweise dem Bromid oder Jodid vornehmen.

Als pharmakologisch verträgliche Salze werden vor allem Mono-bzw. Dialkali- oder Ammoniumsalze verwendet, die man in üblicher Weise z. B. durch Titration der Verbindungen mit anorganischen oder organischen Basen wie z.B. Natrium- oder Kaliumhydrogencarbonat, Natronlauge, Kalilauge, wässrigem Ammoniak oder Aminen wie z.B. Trimethyl- oder Triethylamin hergestellt werden.

Die Salze werden in der Regel durch Umfällen aus Wasser/Methanol oder Wasser/Aceton gereinigt.

Die erfindungsgemäßen neuen Substanzen der Formel I und ihre Salze können in flüssiger oder fester Form enteral oder parenteral appliziert werden. Hierbei kommen alle üblichen Applikationsformen infrage, beispielsweise Tabletten, Kapseln, Dragees, Sirupe, Lösungen, Suspensionen etc.. Als Injektionsmedium kommt vorzugsweise Wasser zur Anwendung, welches die bei Injektionslösungen üblichen Zusätze wie Stabilisierungsmittel, Lösungsvermittler und Puffer enthält.

Derartige Zusätze sind z.B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung. Flüssige Trägerstoffe für Injektionslösungen müssen steril sein und werden vorzugsweise in Ampullen abgefüllt. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten.

Die Dosierung kann von verschiedenen Faktoren, wie Applikationsweise, Spezies, Alter und/oder individuellem Zustand abhängen. Die tägliche zu verabreichenden Dosen liegen bei etwa 1-1000 mg/Mensch, vorzugsweise bei 10-200 mg/Mensch und können auf einmal oder mehrere Male verteilt eingenommen werden.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden Diphosphonsäuren, sowie deren Natriumsalze, Methyl-, bzw. Ethylester:
Azacyclopentadecan-2,2-diphosphonsäure
Azacyclopentadecan-2,2-diphosphonsäure (Mononatriumsalz)
Azacyclooctadecan-2,2-diphosphonsäure
Azacyclooctadecan-2,2-diphosphonsäure (Mononatriumsalz)

Die nachfolgenden Beispiele zeigen einige der Verfahrensvarianten, die zur Synthese der erfindungsgemäßen Verbindungen verwendet werden können. Sie sollen jedoch nicht eine Einschränkung des Erfindungsgegenstandes darstellen. Die Verbindungen fallen in der Regel als hochschmelzende Festprodukte an (Mono- oder Dinatriumsalz), deren Struktur durch ¹H-, ³¹p-und gegenbenenfalls durch ¹³C-NMR-Spektroskopie gesichert wurde. Die Reinheit der Substanzen wurde mittels C,H,N,P,S, Na-Analyse sowie durch Dünnschichtelektrophorese (Cellulose, Oxalat-Puffer von pH = 4.0) bestimmt.

Die als Ausgangsverbindungen eingesetzten Lactame der allgemeinen Formel II werden nach bekannten Verfahren hergestellt z.B. in dem man die cyclischen Ketone mit Hydroxylamin-O-Sulfonsäure umsetzt.

Die für die Beispiele benötigen Edukte wurden, sofern Sie nicht kommerziell erhältlich waren, in Analogie zur folgenden Literaturstelle synthetisiert: Synthesis 1979 537-538.

### Beispiel 1

### Azacyclononan-2,2-diphosphonsäure

2-Azacyclononanon (700 mg, 5 mmol) und Phosphorigsäure (800 mg, 10 mmol) werden unter Stickstoff 1 Stunde bei 100°C erhitzt. Dann wird Phosphortrichlorid (1 ml, 11 mmol) zugetropft. Nach weiteren 12 Stunden bei 100°C wird abgekühlt, mit 50 ml Wasser versetzt, und 1 Stunde auf 100°C erwärmt. Der entstandene Niederschlag wird abgesaugt und das Filtrat im Vakuum eingeengt. Der Rückstand wird aus Wasser umkristallisiert.

Ausbeute: 800 mg (56 % d.Th.); Smp. 210-211°C.

### Beispiel 2

### Azacyclononan-2.2-diphosphonsäure (Mononatriumsalz)

Azacyclononan-2,2-diphosphonsäure (200 mg, 0.7 mmol) wird in Wasser 20 ml aufgenommen und mit 1 N NaOH auf pH 5 eingestellt. Die Lösung wird gefriergetrocknet.

DC (Cellulose; Essigsäureethylester, Eisessig, Wasser 3:1:1): R_{f}: 0.24

### Beispiel 3

### Azacyclodecan-2.2-diphosphonsäure

2-Azacyclodecanon (460 mg, 3 mmol) und Phosphorigsäure (500 mg, 6 mmol) werden unter Stickstoff in 20 ml Chlorbenzol gelöst und 1 Stunde bei 100°C gerührt. Unter Rühren wird Phosphortrichlorid (0.6 ml, 6.6 mmol) zugetropft. Nach weiteren 10 Stunden bei 100°C wird abgekühlt und das Chlorbenzol sorgfältig abdekantiert. Der Rückstand wird mit 30 ml 6 N Salzsäure versetzt und 6 Stunden am Rückfluß gekocht. Die Mischung wird abgekühlt, der entstandene Niederschlag abgesaugt und das Filtrat im Vakuum eingeengt. Der Rückstand wird aus Wasser/Aceton zur Kristallisation gebracht.

Ausbeute 410 mg (45 % d.Th.), Smp. 286-288°C

### Beispiel 4

### Azacyclodecan-2,2-diphosphonsäure (Mononatriumsalz)

Azacyclodecan-2,2-diphosphonsäure (200 mg, 0.66 mmol) wird analog Beispiel 2 behandelt.

DC (Cellulose; Essigsäureethylester/Eisessig/Wasser 3:1:1): R_{f}: 0.42

### Beispiel 5

### Azacycloundecan-2,2-diphosphonsäure

2-Azacycloundecanon (3.4 g, 20 mmol) und Phosphorigsäure (3.2 g, 40 mmol) werden unter Stickstoff bei 80°C gerührt bis eine homogene Schmelze entstanden ist. Dann wird langsam Phosphorylchlorid (3.82 ml, 41 mmol) zugetropft und 7 Stunden auf 100°C erhitzt. Der entstandene Schaum wird bei Raumtemperatur mit 100 ml Wasser versetzt und 1 Stunde auf 100°C erhitzt. Der entstandene Niederschlag wird abfiltriert und das Filtrat im Vakuum eingeengt. Der Rückstand wird aus Wasser umkristallisiert.
Ausbeute: 4.75 g (76 % d.Th.), Smp. 220-222°C

| | | | | | |
|---|---|---|---|---|---|
| C₁₀H₂₃NO₆P₂: | ber.: | C 38.01 | H 7.35 | N 4.44 | P 19.65 |
| (315.243) | gef.: | 38.08 | 7.33 | 4.46 | 19.90 |

### Beispiel 6

### Azacycloundecan-2,2-diphosphonsäure

2-Azacycloundecanon (1.7 g, 10 mmol) und Phosphorigsäure (1.6 g, 20 mmol) werden unter Stickstoff in 30 ml Chlorbenzol gelöst und 1 Stunde auf 140°C erhitzt. Die Umsetzung mit Phosphortrichlorid und die Aufarbeitung erfolgt analog Beispiel 2.

Der Rückstand wird aus Wasser umkristallisiert. Ausbeute: 1.6 g (51 % d.Th.), Smp. 220-222°C.

### Beispiel 7

### Azacycloundecan-2,2-diphosphonsäure (Mononatriumsalz)

Azacycloundecan-2,2-diphosphonsäure (200 mg, 0.64 mmol) wird analog Beispiel 2 behandelt.

DC (Cellulose; Essigsäureethylester, Eisessig, Wasser 3:1:1): R_{f}: 0.46

### Beispiel 8 (Referenzverbindung)

### Azacyclododecan-2,2-diphosphonsäure

2-Azacyclododecanon (2.2 g, 12 mmol) und Phosphorigsäure (2.0 g 24 mmol) werden unter Stickstoff bei 100°C erhitzt bis eine homogene Schmelze entstanden ist. Die Umsetzung mit Phosphorylchlorid (2.25 ml, 24 mmol) und die Aufarbeitung erfolgt analog zu Beispiel 3.

Ausbeute: 2.3 g (64 % d.Th.), Smp. 188-191°C.

### Beispiel 9

### Azacyclododecan-2.2-diphosphonsäure (Mononatriumsalz)

Azacyclododecan-2,2-diphosphonsäure (200 mg, 0.61 mmol) wird analog Beispiel 2 behandelt.

DC (Cellulose; Essigsäureethylester, Eisessig, Wasser 2:1:1): R_{f}: 0.63

### Beispiel 10

### Azacyclotetradecan-2,2-diphosphonsäure

2-Azacyclotetradecanon (1.1 g, 5 mmol) und Phosphorigsäure (0.8 g, 10 mmol) werden in 10 ml Chlorbenzol unter Stickstoff 1 Stunde auf 80°C erhitzt. Unter Rühren wird Phosphorylchlorid (1 ml, 11 mmol) zugetropft. Nach 10 Stunden bei 100°C wird abdekantiert, der Rückstand mit 40 ml Wasser versetzt und 1 Stunde am Rückfluß gekocht. Der entstandene Niederschlag wird abgesaugt, sorgfältig mit Wasser nachgewaschen und getrocknet.

Ausbeute: 1.1 g (62 % d.Th.), Smp. 218-220°C.

### Beispiel 11

### Azacyclotetradecan-2,2-diphosphonsäure

2-Azacycloetetradecanon (2.1 g, 10 mmol) und Phosphorigsäure (0.8 g, 10 mmol) werden analog zu Beispiel 5 mit Phosphorylchlorid (1 ml, 11 mmol) umgesetzt. Die Aufarbeitung erfolg wie in Beispiel 6.

Ausbeute: 2.78 g (78 % d.Th.), Smp. 218-220°C.

### Beispiel 12

### Azacyclotetradecan-2,2-diphosphonsäure (Mononatriumsalz)

Azacyclotetradecan-2,2-diphosphonsäure (200 mg, 0.56 mmol) wird analog Beispiel 2 behandelt.

DC: (Cellulose; Essigsäureethylester, Eisessig, Wasser 2:1:1): R_{f}: 0.62 Beispiel 13

### Azacyclohexadecan-2.2-diphosphonsäure

2-Azacyclohexadecanon (2 g, 8.4 mmol) und Phosphorigsäure (1.4 g, 16.8 mmol) werden analog Beispiel 3 umgesetzt und aufgearbeitet. Der beim Erhitzen mit Wasser entstandene Niederschlag wird abgesaugt, mit Wasser nachgewaschen, in Methanol suspendiert und erneut abgesaugt.

Ausbeute: 2.4 g (74 % d.Th.), Smp. 215-217°C.

### Beispiel 14

### Azacyclohexadecan-2,2-diphosphonsäure

2-Azacyclohexadecanon (900 mg, 3.8 mmol) und Phosphorigsäure (600 mg, 7.6 mmol) werden analog zu Beispiel 4 umgesetzt. Der beim Abkühlen entstandene Niederschlag wird abgesaugt, mit Wasser nachgewaschen, in Methanol suspendiert und erneut abgesaugt.

### Ausbeute: 880 mg (60 % d.Th.), Smp. 215-217°C

### Beispiel 15

### Azacyclohexadecan-2,2-diphosphonsäure (Mononatriumsalz)

Azacyclohexadecan-2,2-diphosphonsäure (200 mg, 0.52 mmol) wird analog zu Beispiel 2 behandelt.

DC (Cellulose; Essigsäureethylester, Eisessig, Wasser 3:1:1): R_{f}: 0.65

### Beispiel 16

### Azacycloheptadecan-2,2-diphosphonsäure

2-Azacycloheptadecanon (5 g, 20 mmol) und Phosphorigsäure (3.2 g, 40 mmol) werden unter Stickstoff in 10 ml Chlorbenzol gelöst. Bei 5°C wird Phosphortrichlorid (5.2 ml, 60 mmol) während 1 Stunde langsam zugetropft. Dann wird unter Kühlung 1.6 ml Wasser zugetropft und langsam auf 90-100°C erhitzt. Nach 10 Stunden wird abgekühlt und 250 ml Wasser zugegeben. Der entstandene Niederschlag wird abgesaugt, mit viel Wasser nachgewaschen, in Methanol suspendiert und erneut abgesaugt.

Ausbeute: 5.4 g (68 % d.Th.), Smp. 210-212°C.

### Beispiel 17

### Azacycloheptadecan-2,2-diphosphonsäure (Mononatriumsalz)

Azacycloheptadecan-2,2-diphosphonsäure (200 mg, 0.50 mmol) wird analog zu Beispiel 2 behandelt.

DC (Cellulose; Essigsäureethylester, Eisessig, Wasser 3:1:1): R_{f}: 0.67

## Patentansprüche

1. Arzneimittel, enthaltend mindestens eine der Verbindungen der Formel I 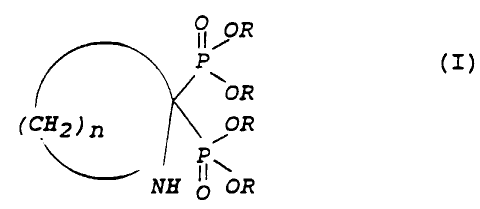 in der
n eine ganze Zahl zwischen 7 und 16 und R Wasserstoff oder C₁-C₄-Alkyl
bedeuten,
sowie deren pharmakologisch verträgliche Salze neben üblichen Träger- und Hilfsstoffen.

2. Verwendung von Verbindungen der Formel I gemäß Anspruch 1 zur Herstellung von Arzneimitteln zur Behandlung von Knochenstoffwechselstörungen.

3. Neue Verbindungen der Formel I' 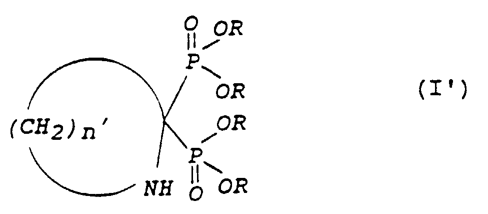 in der
n' eine ganze Zahl zwischen 7 und 16 und
R Wasserstoff oder C₁-C₄-Alkyl
bedeuten,
mit der Maßgabe, daß n' nicht die Zahl 11 darstellen darf,
sowie deren pharmakologisch verträgliche Salze.

4. Verbindungen der Formel I', gemäß Anspruch 3 ausgewählt aus der Gruppe
Azacyclononan-2,2-diphosphonsaure
Azacyclodecan-2,2 diphosphonsäure
Azacycloundecan-2,2-diphosphonsäure
Azacyclotetradecan-2,2-diphosphonsäure
Azacyclopentadecan-2,2-diphosphonsäure
Azacyclohexadecan-2,2-diphosphonsäure
Azacycloheptadecan-2,2-diphosphonsäure
Azacyclooctadecan-2,2-diphosphonsäure
sowie deren pharmakologisch unbedenkliche Salze.

5. Verfahren zur Herstellung von Verbindungen der Formel I' 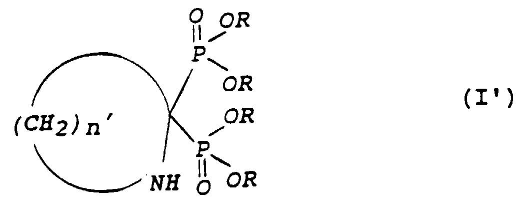 in der
n' eine ganze Zahl zwischen 7 und 16 ist
R Wasserstoff oder C₁-C₄-Alkyl
bedeuten
mit der Maßgabe, daß n' nicht die Zahl 11 darstellen darf,
sowie deren pharmakologisch verträgliche Salze,
dadurch gekennzeichnet, daß man ein Lactam der Formel II 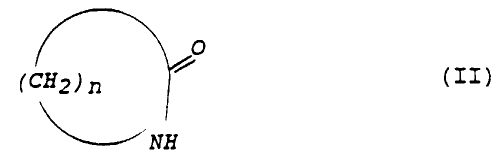 in der n die angegebene Bedeutung hat,
mit einem Gemisch aus phosphoriger Säure oder Phosphonsäure und einem Phosphorhalogenid oder Phosphorylchlorid oder direkt mit einem Phosphorhalogenid in Gegenwart von Wasser zur Reaktion bringt,
und anschließend gewünschtenfalls die erhaltenen Verbindungen in deren Ester oder pharmakologisch unbedenkliche Salze überführt.

## Claims

1. Medicament containing at least one of the compounds of the formula I 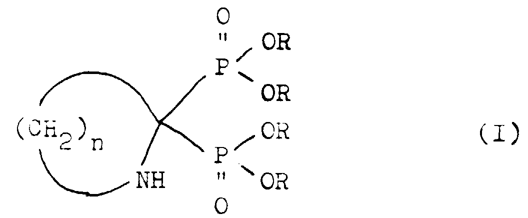 in which n signifies a whole number between 7 and 16 and R hydrogen or C₁-C₄-alkyl, as well as their pharmacologically compatible salts, besides usual carrier ans adjuvent materials.

2. Use of compouds of the formula I according to claim 1 for the preparation of medicaments for the treatment of bone metabolism disturbances.

3. New compounds of the formula I' 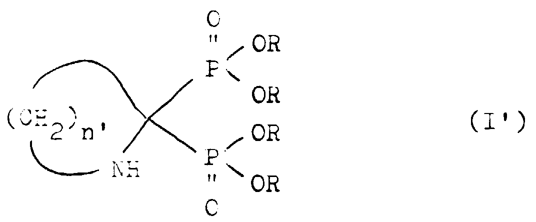 in which n' signifies a whole number between 7 and 16 and R hydrogen or C₁-C₄-alkyl, with the proviso that n' is not to represent the number 11, as well as their pharmacologically compatible salts.

4. Compounds of the formula I' according to claim 3 selected from the group
azacyclononane-2,2-diphosphonic acid
azacyclodecane-2,2-diphosphonic acid
azacycloundecane-2,2-diphosphonic acid
azacyclotetradecane-2,2-diphosphonic acid
azacyclopentadecane-2,2-diphosphonic acid
azacyclohexadecane-2,2-diphosphonic acid
azacycloheptadecane-2,2-diphosphonic acid
azacyclooctadecane-2,2-diphosphonic acid
as well as their pharmacologically compatible salts.

5. Process for the preparation of compounds of the formula I' 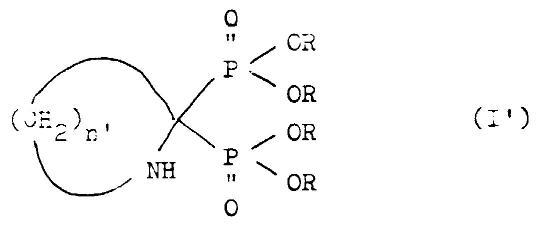 in which n' signifies a whole number between 7 and 16 and R hydrogen or C₁-C₄-alkyl, with the proviso that n' is not to represent the number 11, as well as of their pharmacologically compatible salts, characterised in that one brings a lactam of the formula II 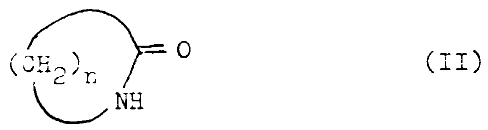 in which n has the given meaning, to reaction with a mixture of phosphorous acid or phosphoric acid and a phosphorus halide or phosphoryl chloride or directly with a phosphorus halide in the presence of water and subsequently, if desired, converts the compounds obtained into their esters or pharmacologically compatible salts.

## Revendications

1. Médicament contenant au moins un des composés de formule I 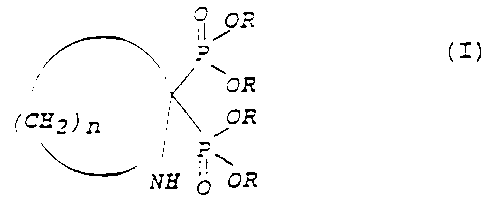 dans laquelle
n représente un nombre entier compris entre 7 et 16 et R représente un atome d'hydrogène ou un groupe alkyle en C₁-C₄,
ainsi que ses sels pharmacologiquement acceptables et des véhicules et adjuvants usuels.

2. Utilisation de composés de formule I selon la revendication 1 pour la préparation de médicaments destinés au traitement de troubles du métabolisme osseux.

3. Nouveaux composés de formule I' 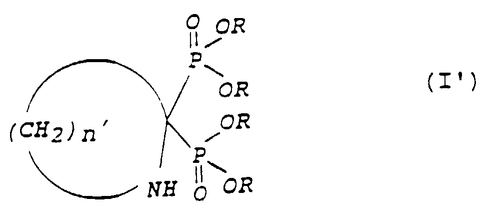 dans laquelle
n' représente un nombre entier compris entre 7 et 16 et
R représente un atome d'hydrogène ou un groupe alkyle en C₁-C₄,
étant entendu que n' ne doit pas représenter le nombre 11, ainsi que leurs sels pharmacologiquement acceptables.

4. Composés de formule selon la revendication 3, choisis dans le groupe constitué par les composés
acide azacyclononane-2,2-diphosphonique
acide azacyclodécane-2,2-diphosphonique
acide azacycloundécane-2,2-diphosphonique
acide azacyclotétradécane-2,2-diphosphonique
acide azacyclopentadécane-2,2-diphosphonique
acide azacyclohexadécane-2,2-diphosphonique
acide azacycloheptadécane-2,2-diphosphonique
acide azacyclooctadécane-2,2-diphosphonique
ainsi que leurs sels pharmacologiquement acceptables.

5. Procédé de préparation de composés de formule I' 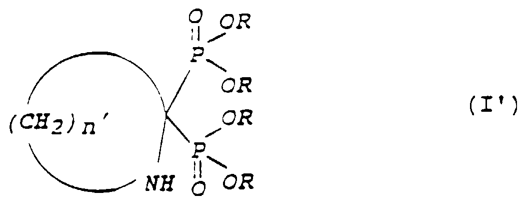 dans laquelle
n' représente un nombre entier compris entre 7 et 16 et
R représente un atome d'hydrogène ou un groupe alkyle en C₁-C₄,
étant entendu que n' ne doit pas représenter le nombre 11,
ainsi que leurs sels pharmacologiquement acceptables,
caractérisé par le fait qu'on fait réagir un lactame de formule II 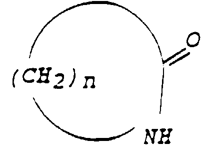 dans laquelle n a la signification mentionnée,
avec un mélange d'acide phosphoreux ou d'acide phosphonique et d'un halogénure de phosphore ou de chlorure de phosphoryle ou directement avec un halogénure de phosphore en présence d'eau,
et ensuite, on transforme éventuellement les composés obtenus en leurs esters ou sels pharmacologiquement acceptables.
